# EUROPÄISCHE PATENTSCHRIFT

(11) **EP 4 450 026 B1**
(45) Veröffentlichungstag und Bekanntmachung des Hinweises auf die Patenterteilung: **10.06.2026**
(21) Anmeldenummer: 23168354.1
(22) Anmeldetag: 17.04.2023
(51) Int. Cl.: A61F 2/12

(54) **KIT-OF-PARTS-WERKZEUGSATZ UND VERFAHREN FÜR DIE HERSTELLUNG VON MIT EINER SCHAUMLAGE VERSEHENEN IMPLANTATHÜLLEN AUF SILIKONBASIS**
KIT-OF-PARTS TOOL KIT AND METHOD FOR PRODUCING SILICONE-BASED IMPLANT SHELLS PROVIDED WITH A FOAM LAYER
KIT D'OUTIL DE PIÈCES ET PROCÉDÉ DE FABRICATION D'ENVELOPPES D'IMPLANT À BASE DE SILICONE POURVUES D'UNE COUCHE DE MOUSSE

(43) Veröffentlichungstag der Anmeldung: 23.10.2024
(73) Patentinhaber: Polytech Health & Aesthetics GmbH, 64807 Dieburg (DE)
(72) Erfinder: Otto, Patrick Christian Josef, 64823 Groß-Umstadt (DE); Smit, Jan Johan Hendrik, 2401 MT Alphen aan den Rijn (NL)
(74) Vertreter: Viering, Jentschura & Partner mbB Patent- und Rechtsanwälte

(56) Entgegenhaltungen:
- WO-A1-2014/093669
- US-B2- 8 679 279

## Beschreibung

Die Erfindung betrifft einen Kit-of-Parts-Werkzeugsatz für die Herstellung von mit einer Schaumlage versehenen Implantathüllen auf Silikonbasis. Die Erfindung betrifft auch ein Verfahren zur Herstellung einer mit einer Schaumlage versehenen Implantathülle auf Silikonbasis.

Aus der WO 2014/093669 A1 gehen eine Vorrichtung und ein Verfahren zur Herstellung eines Brustimplantats mit veränderlicher Oberfläche hervor. Die US 8 679 279 B2 betrifft ein Verfahren zur Herstellung eines texturierten Brustimplantats mit einer Oberflächenstruktur, die durch miteinander verbundene Poren gekennzeichnet ist, umfassend das Aufbringen eines Texturierungsmaterials auf einen Formkern, Auflegen einer Polyurethanschaumplatte auf das Texturierungsmaterial, Bewirken einer Integration zwischen dem Texturierungsmaterial und der Polyurethanschaumplatte durch Pressen der Polyurethanschaumplatte in das Texturierungsmaterial, Erzeugen eines Verbundstoffs aus gehärtetem Texturierungsmaterial und Polyurethanschaum durch Härten des Texturierungsmaterials, Entfernen des Polyurethanschaums von dem ausgehärteten Texturierungsmaterial durch Auftragen eines Polyurethanlösungsmittels auf den Verbundstoff.

Es ist eine Aufgabe der Erfindung, einen Kit-of-Parts-Werkzeugsatz und/oder ein Verfahren für die Herstellung von mit einer Schaumlage versehenen Implantathüllen auf Silikonbasis bereitzustellen, das die Nachteile des Stands der Technik überwindet, insbesondere einfach und rasch verwendbar ist, hohen Hygiene- und/oder Qualitätsanforderungen genügt, geeignet ist, um Implantathüllen, insbesondere Brustimplantathüllen, einheitlich mit hoher Präzision herzustellen und/oder Beschädigungen und andere Mängel bei der Herstellung zu vermeiden. Diese Aufgabe wird gelöst durch den Gegenstand der unabhängigen Ansprüche.

Gemäß einem ersten Aspekt der Erfindung ist ein Kit-of-Parts-Werkzeugsatz für die Herstellung von mit einer Schaumlage versehenen Implantathüllen auf Silikonbasis vorgesehen, der eine Implantathüllenfertigungsform, mindestens eine Haltevorrichtung, eine Auflageeinheit, eine Fixiervorrichtung sowie eine Ringstruktur umfasst.

Der Kit-of-Parts-Werkzeugsatz gemäß dem ersten Aspekt umfasst eine Implantathüllenfertigungsform mit einer Vorderseite, einer Rückseite und einem Übergang von Vorder- zur Rückseite sowie mit einer rückseitig vorliegenden Anbindungseinheit für eine Haltevorrichtung, insbesondere einen Haltestab.

Gemäß dem ersten Aspekt der Erfindung umfasst der Kit-of-Parts-Werkzeugsatz mindestens eine Haltevorrichtung, insbesondere mindestens einen Haltestab, mit einem ersten Ende und einem dem ersten Ende gegenüberliegenden zweiten Ende mit einer Längsachse und mit einer zu der Anbindungseinheit der Implantathüllenfertigungsform korrespondierenden Anbindungseinheit am ersten Ende. Insbesondere umfasst die Haltevorrichtung einen Durchgangs- oder Aufnahmekanal, der im Wesentlichen senkrecht zur Längsachse angeordnet ist.

Vorzugsweise durchdringt der Durchgangs- oder Aufnahmekanal die Haltevorrichtung, insbesondere den Haltestab, senkrecht zu deren Längsachse. Die Haltevorrichtung, insbesondere der Haltestab, kann bezüglich der Längsachse im Wesentlichen rotationssymmetrisch sein.

Insbesondere kann der Kit-of-Part-Werkzeugsatz gemäß dem ersten Aspekt der Erfindung mehrere unterschiedliche Haltevorrichtungen, insbesondere mehrere unterschiedliche Haltestäbe, aufweisen, wobei sich vorzugsweise die mehreren unterschiedlichen Haltevorrichtungen des Kit-of-Parts-Werkzeugsatzes hinsichtlich ihres Materials, ihrer Struktur und/oder ihrer Dimensionierung unterscheiden. Optional kann ein Kit-of-Parts-Werkzeugsatz wenigstens eine erste Haltevorrichtung umfassen, die zum ein- oder mehrmaligen Tauchen und/oder Begießen einer mit dieser ersten Haltevorrichtung verbundenen Implantathüllenfertigungsform mit unausgehärtetem Silikon ausgelegt und eingerichtet ist, um eine, insbesondere vollständig vulkanisierte, Silikonbasishülle zu fertigen. Alternativ oder zusätzlich kann ein Kit-of-Parts-Werkzeugsatz wenigstens eine zweite Haltevorrichtung enthalten, die zum ein- oder mehrmaligen Tauchen und/oder Begießen einer mit dieser zweiten Haltevorrichtung verbundenen, die Silikonbasishülle enthaltenden Implantathüllenfertigungsform mit unausgehärtetem Silikon ausgelegt und eingerichtet ist, um hierauf sodann die Schaumlage anzubringen und gegebenenfalls faltenfrei zu spannen. Die erste und/oder zweite Haltevorrichtung kann vorzugsweisedazu ausgelegt und eingerichtet sein, wenigstens einen Aushärtungsschritt zum Bilden einer Implantatbasishülle/Silikonbasishülle bzw. zum Fixieren einer Schaumlage, insbesondere Polyurethanschaumlage, beschädigungsfrei zu unterlaufen. Die erste Haltevorrichtung und die zweite Haltevorrichtung können aus demselben Material oder aus unterschiedlichen Materialien gebildet sein. Die erste Haltevorrichtung und die zweite Haltevorrichtung können aus demselben Material wie die Implantathüllenfertigungsform oder aus einem anderen Material, z.B. aus Metall wie Stahl oder Aluminium gebildet sein.

Demgemäß umfasst der erfindungsgemäße Kit-of-Parts-Werkzeugsatz für die Herstellung von mit einer Schaumlage versehenen Implantathüllen auf Silikonbasis gemäß dem ersten Aspekt
a) eine Implantathüllenfertigungsformmit einer Vorderseite, einer Rückseite und einem Übergang von Vorder- zu Rückseite sowie mit einer rückseitig vorliegenden Anbindungseinheit für eine Haltevorrichtung, insbesondere einen Haltestab,
b) mindestens eine Haltevorrichtung, insbesondere Haltestab, mit einem ersten und einem gegenüberliegenden zweiten Ende mit einer Längsachse und mit einer zu der Anbindungseinheit der Implantathüllenfertigungsform korrespondierenden Anbindungseinheit an dem ersten Ende, insbesondere umfassend einen Durchgangs- oder Aufnahmekanal im Wesentlichen senkrecht zu der Längsachse,
c) eine Auflageeinheit mit einem ersten Endabschnittmit einer umlaufenden Wandfläche und einem gegenüberliegenden zweiten Endabschnitt und mit einer Anlagefläche an dem ersten Endabschnitt, ausgelegt und eingerichtet, um in einem Areal um die rückseitig vorliegende Anbindungseinheit, insbesondere bündig, anlegbar zu sein, wobei die umlaufende Wandfläche des ersten Endabschnitts der Auflageeinheit bei Anlage der Anlagefläche in einem Übergangsbereich zwischen der Rückseite und dem ersten Endabschnitt eine radiale Einbuchtung bildet, insbesondere an dem ersten Endabschnitt an die Rückseite der Implantathüllenfertigungsform einen spitzen Winkel (α) bildet, wobei insbesondere die Auflageeinheitsich am ersten Endabschnitt in Richtung der Anlageflächev erjüngt,
d) eine Fixiervorrichtung, insbesondere Fixierstab, für die temporäre Fixierung der Auflageeinheit an der Rückseite der Implantathüllenfertigungsform,
   insbesondere mit einem Durchmesser, der kleiner ist als der Durchmesser des Durchgangs- oder Aufnahmekanals, und mit einer Länge, die insbesondere gleich oder größer ist als der Durchmesser des zweiten Endabschnitts der Auflageeinheit, und
e) eine in die radiale Einbuchtung einsetzbare oder eingesetzte Ringstruktur.

Hierbei kann die Anlagefläche der Auflageeinheit eine, insbesondere maximale, Erstreckung, insbesondere einen Durchmesser, aufweisen, der kleiner ist als die kleinste Querschnittsweite der Rückseite.

Alternativ ist gemäß einem zweiten Aspekt der Erfindung ein Kit-of-Parts-Werkzeugsatz für die Herstellung von mit einer Schaumlage versehenen Implantathüllen auf Silikonbasis vorgesehen, der eine Implantathüllenfertigungsform mit einer Haltevorrichtung, eine Auflageeinheit, eine Fixiervorrichtung sowie eine Ringstruktur umfasst.

Gemäß dem zweiten Aspekt der Erfindung umfasst der Kit-of-Parts-Werkzeugsatz eine Implantathüllenfertigungsform mit einer Vorderseite, einer Rückseite und einem Übergang von Vorder- zu Rückseite sowie mit einer rückseitig vorliegenden Haltevorrichtung mit einer Längsachse, insbesondere mit einem Haltestab, insbesondere umfassend einen Durchgangs- oder Aufnahmekanal im Wesentlichen senkrecht zu der Längsachse der Haltevorrichtung, insbesondere des Haltestab. Vorzugsweise durchdringt der Durchgangs- oder Aufnahmekanal die Haltevorrichtung, insbesondere den Haltestab, senkrecht zu deren Längsachse. Bei dem Kit-of-Parts-Werkzeugsatz gemäß dem zweiten Aspekt der Erfindung ist die Haltevorrichtung vorzugsweise fest, beispielsweise stoffschlüssig, mit der Implantathüllenfertigungsform verbunden. Insbesondere ist die Haltevorrichtung integral mit der Implantathüllenfertigungsform gebildet. Die Haltevorrichtung, insbesondere der Haltestab, kann bezüglich der Längsachse im Wesentlichen rotationssymmetrisch sein. Die fest mit der Implantathüllenfertigungsform verbundene Haltevorrichtung ist dazu ausgelegt und eingerichtet, die mehreren Schritte des Herstellungsverfahrens zusammen mit der Implantathüllenfertigungsform zu durchlaufen, insbesondere beschädigungsfrei und/oder zerstörungsfrei. Die fest mit der Implantathüllenfertigungsform verbundene Haltevorrichtung kann aus demselben Material wie die Implantathüllenfertigungsform oder aus einem anderen Material, beispielsweise Metall wie Stahl oder Aluminiumgebildet sein.

Demgemäß umfasst der erfindungsgemäße Kit-of-Parts-Werkzeugsatz für die Herstellung von mit einer Schaumlage versehenen Implantathüllen auf Silikonbasis gemäß dem zweiten Aspekt
i) eine Implantathüllenfertigungsform mit einer Vorderseite, einer Rückseite und einem Übergang von Vorder- zu Rückseite sowie mit einer rückseitig vorliegenden Haltevorrichtung mit einer Längsachse, insbesondere einem Haltestab, insbesondere umfassend einen Durchgangs- oder Aufnahmekanal im Wesentlichen senkrecht zu der Längsachse,
ii) eine Auflageeinheit mit einem ersten Endabschnittmit einer umlaufenden Wandfläche und einem gegenüberliegenden zweiten Endabschnitt und mit einer Anlagefläche an dem ersten Endabschnitt, ausgelegt und eingerichtet, um in einem Areal um die rückseitig vorliegende Haltevorrichtung, insbesondere bündig, anlegbar zu sein, wobei die umlaufende Wandfläche des ersten Endabschnitts der Auflageeinheit bei Anlage der Anlagefläche an dem ersten Endabschnitt an die Rückseite der Implantathüllenfertigungsform in einem Übergangsbereich zwischen der Rückseite und dem ersten Endabschnitt eine radiale Einbuchtung bildet, insbesondere einen spitzen Winkel (α) bildet, wobei insbesondere die Auflageeinheitsich am ersten Endabschnitt in Richtung der Anlagefläche verjüngt,
iii) eine Fixiervorrichtung, insbesondere Fixierstab, für die temporäre Fixierung der Auflageeinheit an der Rückseite der Implantathüllenfertigungsform,
   insbesondere mit einem Durchmesser, der kleiner ist als der Durchmesser des Durchgangs- oder Aufnahmekanals, und mit einer Länge, die insbesondere gleich oder größer ist als der Durchmesser des zweiten Endabschnitts der Auflageeinheit, und
iv) eine in die Einbuchtung einsetzbare oder eingesetzte Ringstruktur.

Hierbei kann die Anlagefläche die Auflageeinheit eine, insbesondere maximale, Erstreckung, insbesondere einen Durchmesser, aufweist, der kleiner ist als die kleinste Querschnittsweite der Rückseite.

Gemäß dem ersten oder zweiten Aspekt der Erfindung kann die Implantathüllenfertigungsform ausgelegt und eingerichtet sein zum Herstellen einer Implantathülle, die sich unterbrechungsfrei über sowohl die gesamte Vorderseite wie auch den gesamten Übergang zwischen Vorderseite und Rückseite und die gesamte Rückseite mit Ausnahme des Bereichs der Anbindung der Haltevorrichtung, insbesondere des Zentrums der Rückseite ausdehnt. Vorzugsweise weist die Implantathüllenfertigungsform im Bereich des Übergangs von der Vorder-zur Rückseite eine Querschnittsweite auf. Die Querschnittsweite kann bei einer Implantathüllenfertigungsform mit kreisrundem Querschnitt durch deren konstanten Kreisdurchmesser definiert sein. Im Rahmen der Erfindung sind auch andere Implantathüllenfertigungsformen denkbar, beispielsweise solche mit eiförmigem oder ovalem Querschnitt, wobei eine kleinste Querschnittsweite beispielsweise bei einem ovalen oder eiförmigen Querschnitt durch die kürzeste Diagonale definiert ist, und wobei eine größte Querschnittsweite durch die längste Diagonale definiert ist.

Die Implantathüllenfertigungsform kann, insbesondere als Brustimplantathüllenfertigungsform, symmetrisch, beispielsweise spiegelsymmetrisch (falls eine beispielsweise eiförmige oder ovale Querschnittsform des Implantats vorgesehen ist) oder rotationssymmetrisch sein (falls das Implantat eine kreisförmige Querschnittsform hat). Die Implantathüllenfertigungsform kann, insbesondere auch als Brustimplantathüllenfertigungsform, anatomisch oder tropfenförmig ausgebildet sein.

Der Kit-of-Parts-Werkzeugsatz umfasst sowohl gemäß dem ersten wie auch gemäß dem zweiten Aspekt der Erfindung eine Auflageeinheit mit einem ersten Endabschnitt mit einer umlaufenden Wandfläche und einem gegenüberliegenden zweiten Endabschnitt. Die Auflageeinheit hat an dem ersten Endabschnitt eine Anlagefläche. Die Anlagefläche ist ausgelegt und eingerichtet, um ein Areal an der Rückseite rings um die rückseitig vorliegende Anbindungseinheit und/oder Haltevorrichtung, insbesondere bündig, anlegbar zu sein. Die Anlagefläche hat eine, insbesondere maximale, Erstreckung, insbesondere einen Durchmesser, der kleiner ist als die kleinste Querschnittsweite der Rückseite. Vorzugsweise ist die Anlagefläche kreisförmig. Die Erstreckung kann bei einer kreisförmigen Anlagefläche deren konstanten Außendurchmesser entsprechen. Die Anlagefläche kann eine andere ringartige Form aufweisen, beispielsweise oval, eiförmig, oder polygonal mit abgerundeten Ecken sein, wobei die Erstreckung der Anlagefläche eine minimale Erstreckung entsprechend einer kleinsten Diagonalen der ringartigen Form und eine maximale Erstreckung entsprechend einer größten Diagonalen der ringartigen Form aufweisen kann. Es kann bevorzugt sein, dass die maximale Erstreckung der Anlagefläche kleiner ist als die kleinste Querschnittsweite der Implantathüllenfertigungsform, insbesondere deren Rückseite. Alternativ kann vorgesehen sein, dass die ringartige Form entsprechend der Querschnittsform der Implantathüllenfertigungsform ausrichtbar oder ausgerichtet ist, wobei jeweils in derselben Querrichtung senkrecht zur Längsachse die Erstreckung der ringartigen Form der Anlagefläche kleiner ist als die Querschnittsweite der Implantathüllenfertigungsform. Beispielsweise können in einer ersten Radialrichtung bezüglich der Längsachse die größte Querschnittsweite und eine maximale Erstreckung, die kleiner ist als die größte Querschnittsweite, und in einer zweiten Radialrichtung eine kleinste Querschnittsweite und eine minimale Erstreckung, die kleiner ist als die kleinste Querschnittsweite, ausgerichtet sein, wobei insbesondere die kleinste Querschnittsweite größer, kleiner oder gleich der größten Erstreckung sein kann.

Die die umlaufende Wandfläche des ersten Endabschnitts der Auflageeinheit bildet bei Anlage der Anlagefläche an dem ersten Endabschnitt an der Rückseite der Implantathüllenfertigungsform in einem Übergangsbereich zwischen der Rückseite und dem ersten Endabschnitt eine radiale Einbuchtung bildet, insbesondere einen spitzen Winkel. Vorzugsweise erstreckt sich die, insbesondere bezüglich der Radialrichtung konkave, Einbuchtung in der Umfangsrichtung vollumfänglich um die Mittelachse der Auflageeinheit und/oder die Längsachse der Implantathüllenfertigungsform. Insbesondere ist in einem Zustand, in welchem die Auflageeinheit mit der Anlagefläche an die Rückseite der Implantathüllenfertigungsform angelegt ist, insbesondere indirekt mit wenigstens einer zwischen der Auflageeinheit und der Implantathüllenfertigungsform vorliegenden Silikonbasishülle oder dergleichen, ist in dem Übergangsbereich zwischen der Rückseite und dem ersten Endabschnitt eine Einbuchtung geschaffen, die beispielsweise eine Rinne, Kerbe oder Ringnut beschreiben kann. Es sei klar, dass der spitze Winkel in einer radialen Querschnittsebene zwischen der Rückseite der Implantathüllenfertigungsform und der Außenseite der Auflageeinheit an deren ersten, an die Anlagefläche angrenzenden Endabschnitt, aufgespannt sein kann. Es kann zweckmäßig sein, dass der spitze Winkel nicht mehr als 80°, insbesondere nicht mehr als 70°, vorzugsweise nicht mehr als 60°, beträgt. Zusätzlich oder alternativ kann es zweckmäßig sein, dass der spitze Winkel wenigstens 30°, insbesondere wenigstens 40°, vorzugsweise wenigstens 50°, beträgt. In der Umfangsrichtung kann der spitze Winkel vollumfänglich in einem Bereich zwischen 30° und 80°, insbesondere zwischen 40° und 70°, vorzugsweise zwischen 50° und 60°, liegen. Insbesondere kann der der spitze Winkel in der Umfangsrichtung konstant sein. Insbesondere verjüngt sich die Auflageeinheit am ersten Endabschnitt in Richtung der Anlagefläche. Beispielsweise kann die Auflageeinheit an ihrem zweiten Endabschnitt zylinderhülsenförmig und in dem an die Anlagefläche angrenzenden ersten Endabschnitt kegelstumpfförmig sein. Die Innenseite der Auflageeinheit kann einen konstanten Innendurchmesser aufweisen, der vorzugsweise entlang der kompletten axialen Erstreckungen der Auflageeinheit, sowohl im ersten Endabschnitt wie auch im zweiten Endabschnitt, konstant ist. Die umlaufende Wandfläche erstreckt sich vollkreisförmig um eine Mittelachse. Vorzugsweise sind die Auflageeinheit und die Haltevorrichtung derart aufeinander abgestimmt, dass die Mittelachse der Auflageeinheit und die Längsachse der Haltevorrichtung zueinander korrespondierend, insbesondere parallel, vorzugsweise kollinear, zueinander anzuordnen oder angeordnet sind. Die Auflageeinheit kann in Bezug auf eine Mittelachse rotationssymmetrisch sein. Bei dem erfindungsgemäßen Kit-of-Parts-Werkzeugsatz ist die Auflageeinheit separat zu der Implantathüllenfertigungsform. Die Auflageeinheit und die Implantathüllenfertigungsform können lösbar miteinander verbunden werden. Es kann bevorzugt sein, dass das erste Ende und/oder das zweite Ende der Auflageeinheit formangepasst an die Rückseite der Implantathüllenfertigungsform gebildet ist.

Der Kit-of-Parts-Werkzeugsatz umfasst sowohl gemäß dem ersten wie auch gemäß dem zweiten Aspekt der Erfindung eine Fixiervorrichtung, insbesondere einen Fixierstab, für die temporäre Fixierung der Auflageeinheit an der Rückseite der Implantathüllenfertigungsform. Insbesondere hat die Fixiervorrichtung, vorzugsweise der Fixierstab, einen Durchmesser, der kleiner ist als der Durchmesser des Durchgangs- oder Aufnahmekanals. Insbesondere hat die Fixiervorrichtung, vorzugsweise der Fixierstab, eine Länge, die gleich oder größer ist als der Außendurchmesser am zweiten Endbereich der Auflageeinheit. Es kann besonders bevorzugt sein, dass die Fixiervorrichtung eine Länge aufweist, die größer ist als die Rückseite der Implantathüllenfertigungsform. Die Fixiervorrichtung, insbesondere der Fixierstab, ist bevorzugt separat von der Haltevorrichtung, insbesondere dem Haltestab, gebildet. Die Fixiervorrichtung ist lösbar mit der Auflageeinheit und/oder der Haltevorrichtung verbindbar oder verbunden. Der Durchgangs- oder Aufnahmekanal und die Fixiervorrichtung sind vorzugsweise aufeinander formabgestimmt, insbesondere weisen der Durchgangs- oder Aufnahmekanal und die Fixiervorrichtung zueinander passende, insbesondere komplementäre und/oder kongruente, Querschnittsflächen auf. Gemäß einer besonders bevorzugten Ausführung sind die Querschnittsflächen des Durchgangs- oder Aufnahmekanals und/oder der Fixiervorrichtung kreisförmig. Alternativ kann der Durchgangs- oder Aufnahmekanal dazu ausgelegt und eingerichtet sein, die Fixiervorrichtung, vorzugsweise den Fixierstab in der Längsachsen-Axialrichtung beweglich aufzunehmen, wobei insbesondere der Durchgangs- oder Aufnahmekanal, wie ein Langloch, eine Längserstreckung in der Längsachsen-Axialrichtung aufweisen kann, die größer ist als die Breitenerstreckung des Durchgangs- oder Aufnahmekanals in der Umfangsrichtung- Der Durchmesser der Fixiervorrichtung beziehungsweise des Durchgangs- oder Aufnahmekanals kann im Allgemeinen als deren beziehungsweise dessen Querschnittsbreite bezeichnet werden. Es ist denkbar, dass ein Kit-of-Parts-Werkzeugsatz, insbesondere gemäß dem ersten Aspekt der Erfindung, mehrere unterschiedliche Fixiervorrichtungen, insbesondere Fixierstäbe, umfasst, die sich hinsichtlich ihres Materials, ihrer Struktur und/oder ihrer Dimensionierung unterscheiden. Bei einem Kit-of-Parts-Werkzeugsatz gemäß dem ersten Aspekt der Erfindung kann es vorgesehen sein, dass eine einzige Fixiervorrichtung, insbesondere ein einziger Fixierstab, für die mehreren Haltevorrichtungen des Kit-of-Parts-Werkzeugsatzes bereitgestellt ist. Alternativ können bei einem Kit-of-Parts-Werkzeugsatz gemäß dem ersten Aspekt der Erfindung für wenigstens zwei der mehreren unterschiedlichen Haltevorrichtungen jeweils korrespondierende, Haltevorrichtungs-individuelle Fixiervorrichtungen vorgesehen sein. Alternativ kann es insbesondere vorgesehen sein, dass die Fixiervorrichtung ein Auge (eine Öffnung) zum Aufnehmen der Haltevorrichtung, insbesondere des Haltestabs, aufweist. Die Haltevorrichtung und das Auge sind vorzugsweise aufeinander formabgestimmt, insbesondere weisen die Haltevorrichtung und das Auge zueinander passende, insbesondere komplementäre und/oder kongruente, Querschnittsflächen auf. Gemäß einer besonders bevorzugten Ausführung sind die Querschnittsflächen der Haltevorrichtung und des Auges kreisförmig. Es kann bevorzugt sein, dass das Auge mit einem Innengewinde und die Haltevorrichtung mit einem zu dem Innengewinde formkomplementären Außengewinde ausgestattet ist. Alternativ kann es bevorzugt sein, dass die Haltevorrichtung, insbesondere der Haltestab, und das Auge gemäß einer Passung, beispielsweise einer Spielpassung oder einer Gleitpassung, aufeinander abgestimmt sind, um eine Linearbewegung sowie gegebenenfalls eine Rotationsbewegung der Fixiervorrichtung, insbesondere des Fixierstabs, in Bezug auf die Längsachse der Haltevorrichtung zu gestatten. Optional kann der Kit-of-Parts-Werkzeugsatz zusätzlich wenigstens ein Befestigungsmittel, wie eine Schrauben- bzw. Gewindemutter, aufweisen, um die Fixiervorrichtung in der Längsachsen-Axialrichtung festzulegen. Insbesondere kann die Haltevorrichtung dazu ausgelegt und eingerichtet sein, mit dem Befestigungsmittel zu kooperieren, wobei beispielsweise die Haltevorrichtung zu dem Innengewinde der Mutter komplementäres Außengewinde aufweisen kann. Es kann zweckmäßig sein, dass der Kit-of-Parts-Werkzeugsatz wenigstens ein Befestigungsmittel enthält, wenn die Fixiervorrichtung relativ zu der Haltevorrichtung, beispielsweise mittels des Auges oder eines Langlochs, in der Längsachsen-Axialrichtung beweglich an der Haltevorrichtung gehalten ist.

Der Kit-of-Parts-Werkzeugsatz umfasst sowohl gemäß dem ersten wie auch gemäß dem zweiten Aspekt der Erfindung eine Ringstruktur, die in die radiale Einbuchtung, insbesondere den spitzen Winkel, einsetzbar oder eingesetzt ist Im Folgenden kann im Allgemeinen mit Ringstruktur eine einteilige oder mehrteilige Komponente des Kit-of-Parts-Werkzeugsatzes bezeichnet sein, die in eine Einbuchtung im Übergangsbereich zwischen der Rückseite und den ersten Endabschnitt einsetzbar oder eingesetzte ist. Im Allgemeinen ist die Ringstruktur in den spitzen Winkel unter Bereitstellung eines radialen Anpressdrucks, insbesondere infolge einer federelastischen Rückstellspannung, eine Zugspannung und/oder einer Klemmspannung der Ringstruktur, anlegbar. Die Ringstruktur kann vorzugsweise dazu ausgelegt und eingerichtet sein, eine Schaumlage oder dergleichen, die über die Implantathüllenfertigungsform und, zumindest im Bereich des ersten Endabschnitts, über die Auflageeinheit gestülpt ist, an dem Kit-of-Parts-Werkzeugsatz zu fixieren. Besonders bevorzugt ist die Ringstruktur dazu ausgelegt und eingerichtet, den Aushärtungsschritt beschädigungsfrei und/oder zerstörungsfrei zu ertragen.

Der erfindungsgemäße Kit-of-Parts-Werkzeugsatz ist dank seines Aufbaus und der geringen Anzahl von Komponenten einfach und rasch verwendbar, und genügt höchsten Hygiene- und/oder Qualitätsanforderungen. Mit dem Kit-of-Parts-Werkzeugsatz gemäß der Erfindung können Implantathüllen, insbesondere Brustimplantathüllen, einheitlich mit hoher Präzision hergestellt werden. Dank der Verwendung des Kit-of-Parts-Werkzeugsatzes gemäß der Erfindung können Beschädigungen und andere Mängel bei der Herstellung konsequent und konsistent vermieden werden.

Bei einer Ausführungsform des Kit-of-Parts-Werkzeugsatzes wölbt sich die Rückseite der Implantathüllenfertigungsform insbesondere zum Zentrum derselben aus, d.h. weist eine Erstreckung weg von der Vorderseite auf. Die Rückseite der Implantathüllenfertigungsform kann allerdings auch flach ausgestaltet sein. Zusätzlich oder alternativ kann die Rückseite der Implantathüllenfertigungsform kegelförmig oder kegelstumpfförmig ausgebildet sein. Die Rückseite kann zumindest abschnittsweise oder vollständig eine geradlinige und/oder konvexe Verjüngung aufweisen. Vorzugsweise ist die Rückseite der Implantathüllenfertigungsform symmetrisch, insbesondere rotationssymmetrisch, bezüglich der Längsachse. Vorzugsweise ist der radiale Verlauf der Rückseite der Implantathüllenfertigungsform gemäß der Verjüngung kontinuierlich und/oder konstant. Die Rückseite der Implantathüllenfertigungsform kann beispielsweise kugelstumpfförmig oder kegelstumpfförmig sein. In Bezug auf eine durch den ringförmigen Übergang definierte Fläche ist das von der Vorderseite bedeckte Volumen der Implantathüllenfertigungsform vorzugsweise wenigstens so groß wie, vorzugsweise größer als, das von der Rückseite bedeckte Volumen der Implantathüllenfertigungsform.

Bei dem Kit-of-Parts-Werkzeugsatz gemäß einer bevorzugten Ausführungsform des ersten und/oder zweiten Aspekts ist die Auflageeinheit jenseits des ersten Endabschnitts im Wesentlichen zylindrisch ausgebildet. Insbesondere weist zweite Endabschnitt einen konstanten Zylinderdurchmesser auf. Der zweite Endabschnitt kann eine Wandfläche aufweisen, die vorzugsweise integral, insbesondere materialeinheitlich, mit dem ersten Endabschnitt gebildet ist. Vorzugsweise ist die Wandfläche im zweiten Endabschnitt umlaufend. Es kann bevorzugt sein, dass ist die Wandfläche im zweiten Endabschnitt geschlossen bzw. frei von radialen Öffnungen ist. Der erste Endabschnitt kann außenseitig kegelstumpfförmig sein. Der erste, kegelstumpfförmige Endabschnitt kann zumindest abschnittsweise oder vollständig eine geradlinige Verjüngung, eine konkave (nach innen gewölbte) Verjüngung oder eine konvexe (nach außen gewölbte) Verjüngung aufweisen. Insbesondere verläuft die Verjüngung von der Anlagefläche zum radial äußeren Ende des ersten Endabschnitts kontinuierlich, vorzugsweise konstant.

Der Kit-of-Parts-Werkzeugsatz umfasst gemäß einer bevorzugten Ausführungsform des ersten und/oder zweiten Aspekts eine Ringstruktur, die gebildet ist durch eine federelastische Ringstruktur oder einen reversibel öffenbaren Metallring oder einen biegbaren Draht, der zum Ausbilden einer Ringstruktur ausgelegt und eingerichtet ist. Gemäß einer ersten Alternative ist die Ringstruktur federelastisch. Insbesondere ist die federelastische Ringstruktur bandförmig. Es kann bevorzugt sein, dass die federelastische Ringstruktur aus einem Kunststoffmaterial, einem PET, wie PET-BO oder BO-PET (Mylar), gebildet ist. Vorzugsweise ist die federelastische Ringstruktur eingerichtet und ausgelegt, um zerstörungsfrei über die Implantathüllenfertigungsform führbar und unter Spannung, insbesondere federelastische Rückstellspannung, in den spitzen Winkel zwischen der Rückseite und dem ersten Endabschnitt Auflageeinheit anlegbar zu sein. Gemäß einer zweiten Alternative kann ein reversibel öffenbaren Metallring vorgesehen sein, der dazu eingerichtet und ausgelegt ist, um in den spitzen Winkel zwischen der Rückseite und dem ersten Endabschnitt der Auflageeinheit unter Ausbildung einer Ringstruktur anlegbar zu sein. Gemäß einer weiteren Alternative kann ein biegbarer Draht vorgesehen sein, der dazu eingerichtet und ausgelegt ist, um in den spitzen Winkel zwischen der Rückseite und den ersten Endabschnitt der Auflageeinheit unter Ausbildung einer Ringstruktur anlegbar zu sein.

Bei einer Ausführung des Kit-of-Parts-Werkzeugsatzes gemäß dem ersten Aspekt der Erfindung liegt die rückseitig vorliegende Anbindungseinheit für die Haltevorrichtung im Zentrum der Rückseite der Implantathüllenfertigungsform vor. Es kann bevorzugt sein, dass die Anbindungseinheit für die Haltevorrichtung auf einer gemeinsamen Längsachse der Haltevorrichtung und der Implantathüllenfertigungsform angeordnet ist. Sowohl die Haltevorrichtung als auch die Implantathüllenfertigungsform können in Bezug auf eine gemeinsame Längsachse im Wesentlichen rotationssymmetrisch geformt sein.

Gemäß einer Ausführung des Kit-of-Parts-Werkzeugsatzes gemäß dem ersten Aspekt der Erfindung sind die rückseitig vorliegende Anbindungseinheit für die Haltevorrichtung und die Anbindungseinheit am ersten Ende der Haltevorrichtung dazu ausgelegt und eingerichtet, eine reversibel lösbare kraftschlüssige und/oder formschlüssige Verbindung einzugehen. Die Haltevorrichtung und die Implantathüllenfertigungsform können eine aufeinander abgestimmte Gewindepaarung umfassen. Es kann bevorzugt sein, dass die rückseitig vorliegende Anbindungseinheit für die Haltevorrichtung eine Gewindebohrung umfasst oder darstellt und dass die korrespondierende Anbindungseinheit an dem ersten Ende der Haltevorrichtung ein, insbesondere passgenaues, Gewinde umfasst oder darstellt. Alternativ kann es bevorzugt sein, dass die rückseitig vorliegende Anbindungseinheit für die Haltevorrichtung ein Gewinde umfasst oder darstellt und dass die korrespondierende Anbindungseinheit an dem ersten Ende der Haltevorrichtung eine, insbesondere passgenaue, Gewindebohrung umfasst oder darstellt. Die Anbindungseinheit der Haltevorrichtung und/oder für die Haltevorrichtung kann einen Innendurchmesser, insbesondere ein Gewindedurchmesser, von wenigstens 8 mm, insbesondere wenigstens 10 mm, vorzugsweise wenigstens 12 mm, und/oder nicht mehr als 22 mm, insbesondere nicht mehr als 20 mm, vorzugsweise nicht mehr als 16 mm oder 15 mm, aufweisen die Anbindungseinheit der Haltevorrichtung und/oder für die Haltevorrichtung kann eine axiale Höhe beziehungsweise Tiefe von wenigstens 10 mm, insbesondere wenigstens 15 mm, vorzugsweise wenigstens 20 mm, und/oder nicht mehr als 60 mm, insbesondere nicht mehr als 40 mm, vorzugsweise nicht mehr als 30 mm, aufweisen. Es hat sich herausgestellt, dass eine derartige Anbindungseinheit einfach in der Handhabung ist und besonders gut dazu geeignet ist, einen stabilen Halt für die Implantathüllenfertigungsform an der Haltevorrichtung, insbesondere dem Haltestab, bereitzustellen, insbesondere bei axialen Verlagerungen der Haltevorrichtung, insbesondere des Haltestab, in Bezug auf die Implantathüllenfertigungsform, beispielsweise zum temporären Fixieren der Haltevorrichtung an der Implantathüllenfertigungsform und/oder zum Spannen der Schaumlage auf der Implantathüllenfertigungsform.

Bei einer Ausführung des Kit-of-Parts-Werkzeugsatzes umfasst die Implantathüllenfertigungsform Metall, insbesondere Aluminium, oder Kunststoff, insbesondere Polyacetale, bevorzugt Polyoxymethylen, Polyethersulfone, Polysulfone, Polyphenylsulfone, Polyaryletherketone, insbesondere Polyetheretherketone, Polyetherketone, thermoplastische Polyimide, Polyphenylensulfide, Polyamide, Polyphenylenether, Polycarbonate, Polyethylenterephthalat und Polybutylenterephthalat umfasst oder besteht hieraus. Alternativ oder zusätzlich umfasst die Auflageeinheit Metall, insbesondere Aluminium, oder Kunststoff, insbesondere Polyacetale, bevorzugt Polyoxymethylen, Polyethersulfone, Polysulfone, Polyphenylsulfone, Polyaryletherketone, insbesondere Polyetheretherketone, Polyetherketone, thermoplastische Polyimide, Polyphenylensulfide, Polyamide, Polyphenylenether, Polycarbonate, Polyethylenterephthalat und Polybutylenterephthalat umfasst oder besteht hieraus. Weiter zusätzlich oder alternativ umfasst die Haltevorrichtung und/oder die Fixiervorrichtug, und/oder gegebenenefalls das Befestigungsmittel Metall, insbesondere Aluminium, oder Kunststoff, insbesondere Polyacetale, bevorzugt Polyoxymethylen, Polyethersulfone, Polysulfone, Polyphenylsulfone, Polyaryletherketone, insbesondere Polyetheretherketone, Polyetherketone, thermoplastische Polyimide, Polyphenylensulfide, Polyamide, Polyphenylenether, Polycarbonate, Polyethylenterephthalat und Polybutylenterephthalat umfasst oder besteht hieraus. Die Implantathüllenfertigungsform, die Haltevorrichtung, die Auflageeinheit, die Fixiervorrichtung sowie gegebenenfalls das Befestigungsmittel können unterschiedliche Materialien umfassen oder daraus bestehen, oder paarweise oder alle die gleichen Materialien oder das gleiche Material umfassen oder daraus bestehen. Als Metall für eine oder mehrere Komponenten des Kit-of-Parts-Werkzeugsatzes ist Aluminium einschließlich Aluminiumlegierungen bevorzugt. Als Kunststoff für eine oder mehrere Komponenten des Kit-of-Parts-Werkzeugsatzes sind Polyacetale, insbesondere Polyoxymethylen, oder Polymercompounds oder Polymerblends umfassend wenigstens 50% Polyacetale, insbesondere wenigstens 50% Polyoxymethylen, bevorzugt.

Gemäß einer besonders bevorzugten Ausführung stellt die Implantathüllenfertigungsform eine Brustimplantathüllenfertigungsform dar.

Die Erfindung betrifft auch ein Verfahren zur Herstellung einer mit einer Schaumlage versehenen Implantathülle auf Silikonbasis. Das Verfahren umfasst, in dieser Reihenfolge, die Schritte:
A) Zurverfügungstellung eines Kit-of-Parts-Werkzeugsatzes wie oben beschrieben, insbesondere gemäß dem ersten oder zweiten Aspekt der Erfindung, und
B) ein- oder mehrmaliges Tauchen der mit der mindestens einen Haltevorrichtung verbundenen Implantathüllenfertigungsform in oder Begießen der mit der mindestens einen Haltevorrichtung verbundenen Implantathüllenfertigungsform mit unausgehärtetem flüssigen Silikon oder mit in einem Lösungsmittel gelösten oder dispergierten unausgehärtetem Silikon, so dass die Vorderseite, der Übergang von Vorder- zur Rückseite und die Rückseite bis zu der Haltevorrichtung mit unausgehärtetem Silikon bedeckt wird,
C) vorzugsweise Unterziehen der Bedeckung mit unausgehärtetem Silikon gemäß Schritt B) mindestens einem Aushärtungsschritt, um eine vollständige oder teilweise Vulkanisierung zu erreichen, unter Ausbildung einer Silikonbasishülle,
D) Fixieren der Auflageeinheit dem ersten Endabschnitt an der Rückseite der auf der Implantathüllenfertigungsform vorliegenden, insbesondere vulkanisierten, Silikonbasishülle, so dass die Anlagefläche an dem ersten Endabschnitt, insbesondere flüssigkeitsdicht, zur Anlage gelangt, insbesondere umfassend das Durchführen oder Einlegen der Fixiervorrichtung, insbesondere des Fixierstabs, durch den bzw. in den Durchgangs- oder Aufnahmekanal,
E) Aufbringen mindestens einer unausgehärteten Silikonschicht auf die Silikonbasishülle erhalten gemäß Schritt D) bis zur Auflageeinheit durch ein- oder mehrmaliges Tauchen der Implantathüllenfertigungsform in oder Begießen der Implantathüllenfertigungsform mit unausgehärtetem flüssigen Silikon oder mit in einem Lösungsmittel gelösten oder dispergierten unausgehärtetem Silikon,
F) Anbringen einer Schaumlage, insbesondere einer Polyurethanschaumlage, auf die Lage aus unausgehärtetem Silikon gemäß Schritt E) an die Vorderseite, den Übergang von Vorder- zur Rückseite und den Bereich der Rückseite, der mit unausgehärtetem Silikon bedeckt ist, wobei Material der Schaumlage, das über den Bereich der Rückseite, der mit unausgehärtetem Silikon bedeckt ist, hinausgeht, durch die Ringstruktur in eine Einbuchtung in einem Übergangsbereich, insbesondere den spitzen Winkel, zwischen der Rückseite und dem ersten Endabschnitt der Auflageeinheit angelegt und fixiert wird, und
G) Unterziehen des in Schritt F) erhaltenen Produkts zumindest einem Aushärtungsschritt, um das unausgehärtete Silikon gemäß Schritt B) und E) oder gemäß Schritt E) zu vulkanisieren und um die Schaumlage mit der Implantathülle zu verbinden;

Alternativ umfasst das Verfahren, in dieser Reihenfolge, die Schritte:
A') Zurverfügungstellung eines Kit-of-Parts-Werkzeugsatzes wie oben beschrieben, insbesondere gemäß dem ersten oder zweiten Aspekt der Erfindung, und
B') Zurverfügungstellung einer Implantathüllenfertigungsform, aufweisend eine an der Vorderseite, im Übergang von Vorder- zu Rückseite und an der Rückseite vorliegende, insbesondere vulkanisierte, Silikonbasishülle,
C') Fixieren des ersten Endabschnitts der Auflageeinheit an der Rückseite der auf der Implantathüllenfertigungsform vorliegenden, insbesondere vulkanisierten, Silikonbasishülle, so dass die Anlagefläche an dem ersten Endabschnitt, insbesondere flüssigkeitsdicht, zur Anlage gelangt, insbesondere umfassend das Durchführen oder Einlegen der Fixiervorrichtung, insbesondere des Fixierstabs, durch den bzw. in den Durchgangs- oder Aufnahmekanal,
D') Aufbringen mindestens einer unausgehärteten Silikonschicht auf die Silikonbasishülle erhalten gemäß Schritt C') bis zur Auflageeinheit durch ein- oder mehrmaliges Tauchen der Implantathüllenfertigungsform in oder Begießen der Implantathüllenfertigungsform mit unausgehärtetem flüssigen Silikon oder mit in einem Lösungsmittel gelösten oder dispergierten unausgehärtetem Silikon,
E') Anbringen einer Schaumlage, insbesondere einer Polyurethanschaumlage, auf die Lage aus unausgehärtetem Silikon gemäß Schritt D') an die Vorderseite, den Übergang von Vorder- zur Rückseite und den Bereich der Rückseite, der mit unausgehärtetem Silikon bedeckt ist, wobei Material der Schaumlage, das über den Bereich der Rückseite, der mit unausgehärtetem Silikon bedeckt ist, hinausgeht, durch die Ringstruktur in einer Einbuchtung in einem Übergangsbereich, insbesondere den spitzen Winkel, zwischen der Rückseite und dem ersten Endabschnitt der Auflageeinheit angelegt und fixiert wird, und
F') Unterziehen des in Schritt E') erhaltenen Produkts zumindest einem Aushärtungsschritt, um das unausgehärtete Silikon gemäß Schritt B') und E') oder gemäß Schritt E') zu vulkanisieren und um die Schaumlage mit der Implantathülle zu verbinden.

Die Alternativen erfindungsgemäßen des Verfahren zur Herstellung einer mit einer Schaumlage versehenen Implantathülle auf Silikonbasis unterscheiden sich im Wesentlichen nur dadurch, dass gemäß der ersten Alternative eine Silikonbasisschicht auf der Implantathüllenfertigungsform des Kit-of-Parts-Werkzeugsatzes hergestellt und unter Verwendung zumindest der Implantathüllenfertigungsform und wenigstens einer Haltevorrichtung oder der fest mit der Implantathüllenfertigungsform verbundenen Haltevorrichtung aufgetragen und wenigstens einem Aushärtungsschritt unterzogen wird, wohingegen gemäß der zweiten Alternative eine Implantathüllenfertigungsform mit darauf platzierter Silikonbasishülle bereitgestellt wird. Gemäß dem der zweiten Alternative kann die Silikonbasishülle optional mit einem anderen Werkzeug, welches insbesondere nicht Teil des erfindungsgemäßen Kit-of-Parts-Werkzeugsatzes ist, hergestellt worden sein und anschließend auf der Implantathüllenfertigungsform gehaltert werden

Vorzugsweise wird in dem Schritt (D) beziehungsweise (C') wird die Anlagefläche am ersten Endabschnitt der Auflageeinheit gegen die Rückseite der Implantathüllenfertigungsform sowie vorzugsweise gegen die dort angeordnete Silikonbasishülle gedrückt. Insbesondere wird in dem Schritt (D) beziehungsweise (C') die Auflageeinheit dazu verwendet, das Zentrum der Rückseite der Implantathüllenfertigungsform freizuhalten von der wenigstens einen zusätzlichen unausgehärteten Silikonschicht, welche über die Implantatbasishülle aufgetragen wird. In dem Schritt (D) beziehungsweise (C') werden vorzugsweise mehrere zusätzliche unausgehärtete Silikonschichten, insbesondere wenigstens zwei, vorzugsweise wenigstens vier, besonders bevorzugt wenigstens sechs, und/oder nicht mehr als 20, insbesondere nicht mehr als zehn, vorzugsweise nicht mehr als acht, zusätzliche unausgehärtete Silikonschichten über die Implantatbasishülle aufgetragen.

In Schritt (F) beziehungsweise (E') kann die Schaumlage, die auch als Schaumstofflage bezeichnet sein kann, vorzugsweise zunächst über die Vorderseite, dann um den Übergang herum und schließlich entlang der Rückseite und über den ersten Endabschnitt der Auflageeinheit gezogen werden. Es kann bevorzugt sein, dass die Schaumlage beim Aufziehen über die Implantathüllenfertigungsform, insbesondere mittels der Haltevorrichtung und/oder der Fixiervorrichtung, mit einer Zugspannung beaufschlagt wird. Beispielsweise kann die Schaumlage beim Aufziehen über die Implantathüllenfertigungsform mit einer Zugspannung beaufschlagt werden, indem die Fixiervorrichtung mit der Schaumlage verbunden und entlang der Haltevorrichtung von der Implantathüllenfertigungsform entfernt wird. Die Fixiervorrichtung kann von der Implantathüllenfertigungsform entfernt werden, indem beispielsweise eine Anbindungseinheit, wie eine Gewindepaarung, betätigt wird, oder indem die Fixiervorrichtung, beispielsweise mithilfe eines Befestigungsmittels, betätigt wird.

Gemäß einer bevorzugten Ausführung umfasst das Verfahren ferner als Schritt (H) bzw. Schritt (G') das Entfernen, insbesondere das Abtrennen, von überschüssiger Schaumlage, die durch den Aushärtungsschritt in Schritt (G) oder (F') nicht fest, insbesondere nicht materialschlüssig, mit er Silikonbasishülle verbunden wurde.

Optional kann gemäß einer Ausführung das Verfahren außerdem in einem Schritt (I) bzw. (H') das Entfernen der Implantathülle von der Implantathüllenfertigungsform umfassen. Das Entfernen der Implantathülle von der Implantathüllenfertigungsform kann gemäß einem Schritt (I) bzw. (H') vor dem Schritt (H) bzw. (G') oder, bevorzugt, nach dem Schritt (H) bzw. (G') durchgeführt werden.

Es kann bevorzugt sein, dass Schritt (F) bzw. (E') umfasst, dass als Ringstruktur eine federelastische, insbesondere bandförmige, Ringstruktur oder ein reversibel öffenbarer Metallring oder ein zu einer Ringstruktur biegbarer Draht in die Einbuchtung angelegt und fixiert wird.

Weitere Eigenschaften, Merkmale und Vorteile der Erfindung werden durch die folgende Beschreibung einer bevorzugten Ausführungsform der Erfindung anhand der beiliegenden Zeichnung deutlich, in denen zeigt:
- Figur 1: eine schematische Schnittansicht einer Implantathüllenfertigungsform mit daran befestigter Haltevorrichtung;
- Figur 2: eine schematische Seitenansicht der Komponenten gemäß Figur 1 mit einer Silikonbasishülle auf der Implantathüllenfertigungsform;
- Figur 3: eine schematische Schnittansicht eines erfindungsgemäßen Kit-of-Parts-Werkzeugsatzes;
- Figur 4: eine schematische Seitenansicht des Kit-of-Parts-Werkzeugsatzes gemäß Figur 3;
- Figur 5: eine schematische Schnittansicht des Kit-of-Parts-Werkzeugsatzes gemäß Figur 3 mit einer zusätzlichen unausgehärteten Silikonschicht auf der Implantathüllenfertigungsform;
- Figur 6: eine schematische Seitenansicht des Kit-of-Parts-Werkzeugsatzes gemäß Figur 5;
- Figur 7: eine schematische Schnittansicht des Kit-of-Parts-Werkzeugsatzes gemäß Figur 3 mit einer über die Implantathüllenfertigungsform und die Auflageeinheit gezogenen Schaumlage; und
- Figur 8: eine schematische Seitenansicht des Kit-of-Parts-Werkzeugsatzes mit einer mit einer Schaumlage versehenen Implantathülle auf Silikonbasis.

Es sei klar, dass der im Folgenden anhand der Figuren exemplarische dargestellte erfindungsgemäße Kit-of-Parts Werkzeugsatz 1, die insbesondere zur Verwendung in einem erfindungsgemäßen Verfahren ausgelegt und eingerichtet ist, in der vorliegenden Offenbarung nur schematisch dargestellt und exemplarisch beschrieben wird. Im Rahmen der Offenbarung sind zahlreiche Variationen gegenüber der exemplarisch in den Figuren dargestellten bevorzugten Ausführungsform denkbar.

Mit dem Bezugszeichen 1 ist im Allgemeinen ein erfindungsgemäßer Kit-of-Parts-Werkzeugsatz für die Herstellung von mit einer Schaumlage versehenen Implantathülle auf Silikonbasis bezeichnet. Eine Implantathülle, die durch das erfindungsgemäße Herstellungsverfahren beziehungsweise mit dem erfindungsgemäßen Kit-of-Parts-Werkzeugsatz herstellbar ist, wird mit dem Bezugszeichen 100 bezeichnet.

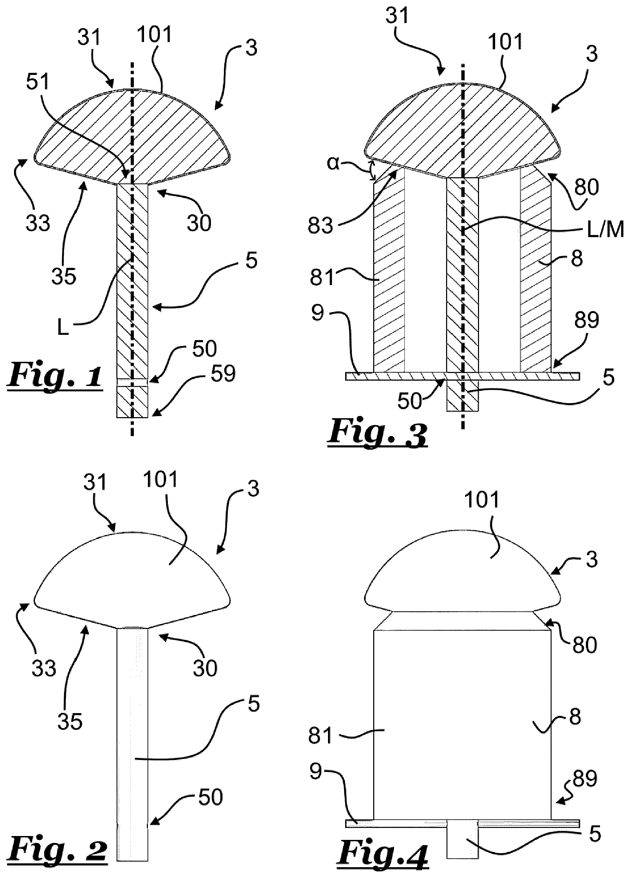

Der beispielsweise in Figur 3 abgebildete erfindungsgemäße Kit-of-Parts-Werkzeugsatz 1 umfasst eine Implantathüllenfertigungsform 3, eine Auflageeinheit 8 und eine Fixiervorrichtung, insbesondere eine Fixierstab 9. Der erfindungsgemäße Kit-of-Parts-Werkzeugsatz 1 kann mindestens eine, vorzugsweise mehrere, Haltevorrichtungen, insbesondere Haltestäbe 5, umfassen, die mit der Implantathüllenfertigungsform 3 lösbar verbunden oder verbindbar sind, oder eine fest, insbesondere integral, mit der Implantathüllenfertigungsform 3 verbundene Haltevorrichtung.

Figur 1 und 2 zeigen die Implantathüllenfertigungsform 3 mit daran befestigter Haltevorrichtung in Form eines Haltestabs 5. Der Haltestabs 5 ist im Wesentlichen rotationssymmetrisch bezüglich Längsachse L. Die Implantathüllenfertigungsform 3 kann bezüglich der Längsachse symmetrisch, beispielsweise spiegelsymmetrisch (falls eine beispielsweise eiförmige oder ovale Querschnittsform des Implantats vorgesehen ist) oder rotationssymmetrisch sein (falls das Implantat eine kreisförmige Querschnittsform hat). Der Haltestab 5 ist im Wesentlichen zylindrisch mit konstantem Durchmesser und einem Durchgangs- oder Aufnahmekanal 50, der den Haltestab 5 senkrecht zur Längsachse L durchdringt. Der Durchgangs- oder Aufnahmekanal 50 ist hinteren dem vorderen Ende 59 des Haltestabs 5 angeordnet. Insbesondere ist der Durchgangs- oder Aufnahmekanal in der hinteren Hälfte, vorzugsweise dem hinteren Viertel, des Haltestabs 5 angeordnet.

Gemäß einem Aspekt kann der Haltestab 5 oder eine andere Haltevorrichtung an seinem vorderen Ende 51 fest, insbesondere stoffschlüssig, mit der Implantathüllenfertigungsform 3 verbunden sein. Es ist denkbar, dass die Haltevorrichtung und die Implantathüllenfertigungsform 3 integral als ein einziges Bauteil gebildet sind. Gemäß einem anderen Aspekt ist am vorderen, ersten Ende 51 des Haltestabs 5 eine Anbindungseinheit vorgesehen und die Implantathüllenfertigungsform 3 hat eine dazu korrespondierende Anbindungseinheit an ihrem Zentrum 30. Die Anbindungseinheiten können beispielsweise eine Gewindepaarung realisieren. Beispielsweise kann die Implantathüllenfertigungsform 3 mit einer Gewindebohrung und der Haltestab 5 mit einem dazu korrespondierenden Gewindebolzen ausgestattet sein. An dem zum ersten Ende 51 gegenüberliegenden zweiten Ende 59 des Haltestabs 5 kann ein Haltegriff zur manuellen Benutzung des Kit-of-Parts-Werkzeugsatzes 1 und/oder eine Befestigungseinrichtung zur Montage des Kit-of-Parts-Werkzeugsatzes an einer Halterung vorgesehen sein.

Die Implantathüllenfertigungsform 3 hat eine Vorderseite 31, die in einem implantierten Zustand die ventrale Seite des Implantats, insbesondere das Brustimplantat, definieren kann und eine zur Vorderseite 31 gegenüberliegende Rückseite 35. Die Implantathüllenfertigungsform 3 weist außerdem einen Übergang 33 von der Vorder-zur Rückseite auf. Senkrecht zu der Längsachse L verläuft eine gedachte Ebene durch den Übergang 33. Es kann bevorzugt sein, dass die gedachte Ebene senkrecht zu der Längsachse L durch die radial äußerste Stelle des Übergangs 33 verläuft.

Vor (auf in Figur 1 über) der gedachten Ebene wölbt sich die Vorderseite 31 konvex und kugelstumpfförmig nach außen. Hinter (in Figur 1 unter) der gedachten Ebene wölbt sich die Rückseite 35 kegelstumpfförmig nach außen. In Bezug auf die gedachte Ebene ist das durch die Vorderseite 31 begrenzte Volumen wesentlich größer als das durch die Rückseite 35 begrenzte Volumen. Die Höhe der Vorderseite 31 in Bezug auf die gedachte Ebene ist größer als die Höhe der Rückseite 35 in Bezug auf die gedachte Ebene. Beginnend an dem Übergang 33 wölbt sich die kugelstumpfförmig Vorderseite 31 wesentlich steiler nach oben als sich beginnend an den Übergang 33 die kegelstumpfförmige Rückseite 35 nach unten neigt. In Bezug die gedachte Ebene kann die aufwärtige Neigung der Vorderseite 31 am Übergang 33 zwischen 45° und 80°, insbesondere zwischen 50° und 85°, vorzugsweise bei etwa 60°, liegen. In Bezug auf die gedachte Ebene kann die abwärtige Neigung der Rückseite 35 am Übergang 33 zwischen 5° und 30°, insbesondere zwischen 10° und 20°, vorzugsweise bei etwa 15°, liegen. Der Übergang 33 ist vorzugsweise als ein, insbesondere konstanter, Umfangsradius der Implantathüllenfertigungsform 3 gebildet.

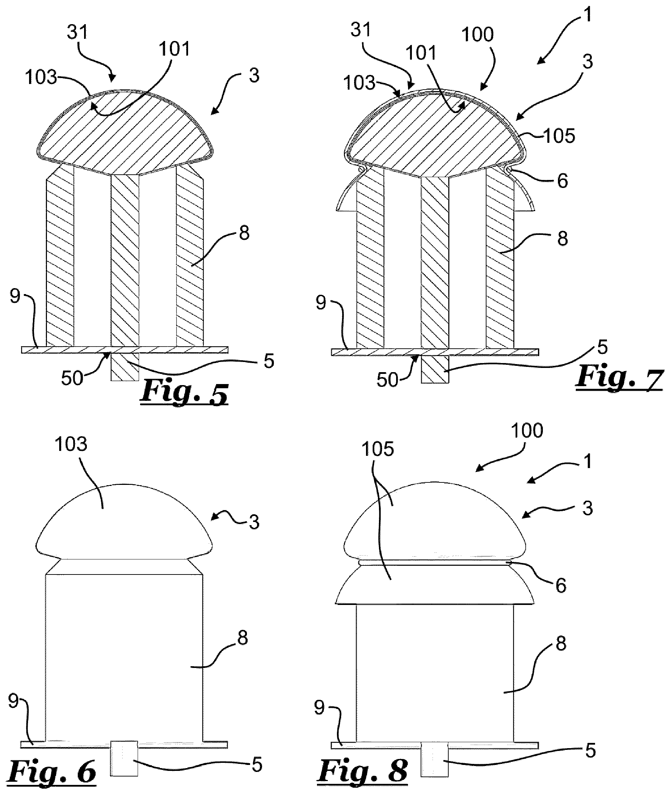

Die Figuren 7 und 8 zeigen den Kit-of-Parts-Werkzeugsatz 1, welcher zusätzlich zu der Implantathüllenfertigungsform 3 und dem Haltestab 5 die in der vorliegenden Ausführung als Fixierstab 9 ausgebildete Fixiervorrichtung und die Auflageeinheit 8 sowie eine Ringstruktur 6 umfasst. In den Figuren 3 bis 8 ist abgebildet, dass die Auflageeinheit 8 durch den Fixierstab 5 an der Rückseite 35 der Implantathüllenfertigungsform 3 gehalten wird.

Die Fixierstab 9 hat in der abgebildeten bevorzugten Ausführung einen geringeren Kreisdurchmesser als Kreisdurchmesser des Haltestabs 5. Der kreisförmige Querschnitt des Fixierstabs 9 ist vorzugsweise formkomplementär zum kreisförmigen Querschnitt des Durchgangs- oder Aufnahmekanals 50. Es sei klar, dass die Fixiervorrichtung, die Haltevorrichtung und/oder dessen optionaler Durchgangs- oder Aufnahmekanal andere Formen als eine Kreisform aufweisen können. Beispielsweise kann der Durchgangs- oder Aufnahmekanal als ein sich in der Längsachsen-Axialrichtung erstreckendes Langloch gebildet sein (nicht näher dargestellt). Alternativ kann beispielsweise die Fixiervorrichtung mit einer Durchgangs- oder Aufnahmeöffnung, wie einem Auge, zum Aufnehmen der Haltevorrichtung geformt sein, wobei insbesondere die Haltevorrichtung frei von einer Durchgangs- oder Aufnahmeöffnung gebildet sein kann (nicht näher dargestellt).

Die Auflageeinheit 8 hat eine umlaufende Wandfläche 81 mit einem ersten Endabschnitt 80 und einem zum ersten Endabschnitt 80 gegenüberliegenden zweiten Endabschnitt 89. Die Auflageeinheit 8 kann eine Mittelachse M aufweisen. Vorzugsweise ist die Auflageeinheit 8 bezüglich der Mittelachse M symmetrisch, insbesondere rotationssymmetrisch. Bei Verwendung des Kit-of-Parts-Werkzeugsatzes 1 kann die Auflageeinheit 8 derart an der Implantathüllenfertigungsform 3 anlegen, dass die Mittelachse M der Auflageeinheit 8 korrespondierend, insbesondere parallel, vorzugsweise (wie abgebildet) kollinear, zu der Längsachse L der Implantathüllenfertigungsform 3 ausgerichtet ist. Bei manchen Ausführungen des erfindungsgemäßen Verfahrens kann es genügen, wenn die Mittelachse M im Wesentlichen korrespondierend zu der Längsachse L angeordnet ist, beispielsweise mit einem Winkelversatz und/oder radialen Versatz im Rahmen einer vorbestimmten Toleranz.

Die Auflageeinheit 8 hat am ersten Endabschnitt 80 einen ersten Außendurchmesser und an dem zweiten Endabschnitt 89 eine zweiten Außendurchmesser. Der zweite Außendurchmesser kann größer sein als der erste Außendurchmesser. Bei der hier abgebildeten Ausführung hat die Auflageeinheit 8 am ersten Endabschnitt 80 und am zweiten Endabschnitt 89 einen konstanten, einheitlichen Innendurchmesser. Die Wanddicke der umlaufenden Wandfläche 81 ist im zweiten Endabschnitt 89 im Wesentlichen konstant. Im ersten Endabschnitt 80 verjüngt sich die Wanddicke.

Figur 3 zeigt, dass die Auflageeinheit 8 am ersten Endabschnitt 80 eine Anlagefläche 83 aufweist. Die Anlagefläche 83 ist korrespondierend zur Rückseite 35 der Implantathüllenfertigungsform 3 geformt, sodass sie bündig daran anliegen kann. Die Auflageeinheit 8 ist vorzugsweise dazu ausgelegt und eingerichtet, mit der Anlagefläche 83 in einen flächigen Ringkontakt mit der Rückseite 35 der Implantathüllenfertigungsform 3 oder einer dort angeordneten Silikonbasishülle 101 bringbar oder gebracht zu sein. Das heißt, die Anlagefläche 83 bildet in Bezug auf die Rückseite 35 eine negativ-Form. Die Anlagefläche 83 kann beispielsweise rotationssymmetrisch sein, wenn die Rückseite 35 rotationssymmetrisch ist. Beispielsweise kann die Anlagefläche 83 eine aufwärts-Neigung aufweisen, die der abwärts-Neigung der Rückseite 35 entspricht.

Wenn die Anlagefläche 83 an die Rückseite 35 angelegt ist, wie beispielsweise in den Figuren 3 und 5 abgebildet, wird außenseitig ein Übergangsbereich zwischen der Implantathüllenfertigungsform 3 und der Auflageeinheit 8 gebildet, der eine radiale Einbuchtung aufweist. Die radiale Einbuchtung kann, wie in dem abgebildeten Beispiel, durch einen spitzen Winkel α zwischen der Anlagefläche 83 und der Außenseite des ersten Endabschnitts 80 gebildet sein. Der spitze Winkel α kann, wie in den Figuren 4 und 6 dargestellt, vollumfänglich konstant sein. Vorzugsweise beträgt der spitze Winkel α wie abgebildet etwa 60°. Die radiale Einbuchtung dient dazu, eine Aufnahme zur sicheren und präzisen sowie reproduzierbaren Positionierung der Ringstruktur 6 bereitzustellen.

Die in den Figuren 7 und 8 dargestellte Implantathülle 100 umfasst im Wesentlichen eine Silikonbasishülle 101, eine Schaumlage 105 und zusätzliche Silikonschichten 103, die die Silikonbasishülle 101 belegen und die Schaumlage 105 zumindest teilweise infiltrieren. Vorzugsweise liegt die Schaumlage 105 auf der Silikonbasishülle 101 auf und dringt nicht in die Silikonbasishülle 101 ein.

Die Implantathülle 100 kann hergestellt werden, indem zunächst der Kit-of-Parts-Werkzeugsatz mit der Silikonbasishülle 101 belegt wird, wobei die Silikonbasishülle 101 alternativ mithilfe zumindest der Implantathüllenfertigungsform 3 des Kit-of-Parts-Werkzeugsatzes 1 geformt und ausgehärtet werden kann oder in einem ausgehärteten Zustand zur weiteren Verwendung mit dem Kit-of-Parts-Werkzeugsatz 1 bereitgestellt wird. Die Figuren 1 und 2 zeigen die mit der Silikonbasishülle 101 belegte Implantathüllenfertigungsform 3, welche mit einem Haltestab 5 verbunden ist.

Zur Herstellung der Implantathülle 100 wird über die Silikonbasishülle 101 wenigstens eine zusätzliche unausgehärtete Silikonschicht 103 aufgetragen. Zu diesem Zweck wird zunächst, wie in den Figuren 3 und 4 abgebildet, ein erster Endabschnitt 80 der Auflageeinheit 8 mit umlaufender Wandfläche 81 gegen die Rückseite 35 und die dortige Silikonbasishülle 101 gedrängt. Dabei wird eine Fixiervorrichtung verwendet, welche gegen das zum ersten Endabschnitt 80 gegenüberliegende zweite Ende 89 der Auflageeinheit 8 drückt. Die Fixiervorrichtung wird an der Haltevorrichtung befestigt. Beispielsweise kann die Fixiervorrichtung als Fixierstab 9 realisiert sein, der in einen passenden Durchgangs- oder Aufnahmekanal 50 des Haltestabs 5 eingesetzt ist. Der vom Haltestab 5 gehaltene Fixierstab 9 wird an zwei diametral gegenüberliegenden Stellen am zweiten Ende 89 der Auflageeinheit 8 angelegt.

Der Abstand zwischen der Fixiervorrichtung zur Implantathüllenfertigungsform 3 ist auf die axiale Höhe der Auflageeinheit 8 abgestimmt. Insbesondere kann der Abstand vom Durchgangs- oder Aufnahmekanal 50 zum ersten Ende 51 der Haltevorrichtung den Abstand der Fixiervorrichtung und der Implantathüllenfertigungsform 3 bestimmen. Optional kann es vorgesehen sein, dass, insbesondere mithilfe der Anbindungseinheiten, der Abstand zwischen der Fixiervorrichtung und der Implantathüllenfertigungsform 3 einstellbar ist. Mithilfe einer Abstandsverstellung kann ein Anpressdruck zum Andrücken der Auflageeinheit 8 an die Rückseite 35 einstellbar sein.

Während die Auflageeinheit 8 mit dem ersten Endabschnitt 80 an der Rückseite 35 und der dort angeordneten Silikonbasishülle 101 vorzugweise abdichtend anliegt, kann nun eine zusätzliche unausgehärtete Silikonschicht 103 über die Silikonbasishülle 101 aufgetragen werden. Es kann zweckmäßig sein, nach dem Aufbringen einzelner zusätzlicher unausgehärteter Silikonschichten einen Zwischenschritt zur Trocknung und/oder teilweisen Aushärtung dieser einzelnen Silikonschicht durchzuführen. Durch die an der Rückseite 35 anliegende Auflageeinheit 8 wird das Zentrum 30 der Rückseite 35 frei von dem zusätzlichen unausgehärteten Silikon gehalten. Dies kann zweckmäßig sein, um zu verhindern, dass die später aufzubringende Schaumlage 105 in einem zentralen Bereich der Rückseite 35 anhaftet. Die wenigstens eine zusätzliche Silikonschicht 103 ist in den Figuren 5 und 6 abgebildet.

Nachdem die wenigstens eine zusätzliche Silikonschicht 103 auf die Implantathüllenfertigungsform 3 aufgebracht worden ist, und bevor diese zusätzliche Silikonschicht 103 vollständig ausgehärtet wird, wird eine Schaumlage 105 über die Implantathüllenfertigungsform 3 gezogen. Dabei wird die Auflageeinheit 8 vorzugsweise nicht von Implantathüllenfertigungsform 3 gelöst. Die Schaumlage 105 wird über die Rückseite 35 der Implantathüllenfertigungsform 3 und den vorderen, ersten Endabschnitt 80 gestülpt.

Dann wird eine Ringstruktur 6 in die radiale Einbuchtung im Übergangsbereich zwischen der Rückseite 35 und den ersten Endabschnitt 80 eingesetzt. Wie oben die Ringstruktur 6 kann beispielsweise, wie abgebildet, als ein federelastisches Band gebildet sein, welches kreisringförmig außenseitig um die Implantathüllenfertigungsform 3 und die Auflageeinheit 8 gelegt wird. Alternativ (nicht näher dargestellt) kann beispielsweise als eines biegbaren Drahts oder eines reversibel öffenbaren Metallrings eine Ringstruktur 6 gebildet werden. Mithilfe der Ringstruktur 6 wird die Schaumlage 105, insbesondere an der Rückseite 35, quer zur Längsachse L beziehungsweise Mittelachse M radial einwärts gegen die wenigstens eine unausgehärtete Silikonlage 103 gedrängt.

Wenn die Ringstruktur 6 als federelastisches Band realisiert ist, kann die Ringstruktur 6 unter Ausnutzung ihrer Elastizität zunächst expandiert werden, um zerstörungsfrei über den übrigen Kit-of-Parts-Werkzeugsatz 1, insbesondere von vorne über die Implantathüllenfertigungsform 3, geführt zu werden. Sodann kann die Ringstruktur 6 unter Spannung in den Übergangsbereich von der Rückseite 35 zum ersten Endabschnitt 80 der Auflageeinheit 8 eingesetzt werden. In dem eingesetzten Zustand der Ringstruktur 6 kann die Spannung ebenfalls unter Ausnutzung der Elastizität der Ringstruktur 6 bereitgestellt werden.

Alternative Ausführungsformen (nicht näher dargestellt), bei denen die Ringstruktur 6, die beispielsweise aus einem Draht oder einem Metallring gebildet sein kann, zunächst geöffnet wird, um in den Übergangsbereich eingesetzt zu werden, und anschließend geschlossen wird, um die Schaumlage 105 zu fixieren, sind ebenfalls denkbar.

Die Schaumlage 105 kann mit der Fixiervorrichtung, insbesondere dem Fixierstab 9, am hinteren Ende der Auflageeinheit 8 fixiert werden. Es kann bevorzugt sein, dass die Schaumlage 105 mit einer Zugspannung beaufschlagt wird. Die Schaumlage 105 kann unter Einwirkung der Ringstruktur 6 mit einer Zugspannung in der Längsachsen-Axialrichtung beaufschlagt werden, damit sich die Schaumlage 105, insbesondere an der Vorderseite 31, dem Übergang 33 und/oder der Rückseite 35, glatt an die Implantathüllenfertigungsform 3 anschmiegt. Durch die Ringstruktur 6 kann eine Haltekraft auf die Schaumlage 105 ausgeübt werden, die, insbesondere während einer Aushärtung, eine Ablösung der Schaumlage 105 von der Implantathüllenfertigungsform 3, insbesondere der unausgehärteten Silikonlage 103 und der Silikonbasishülle 101, entgegenwirkt.

Insbesondere kann es vorgesehen sein, dass der Außendurchmesser der Anlagefläche 83 nicht größer ist, besonders bevorzugt kleiner ist als die kleinste Querschnittsweite der Rückseite 35 der Implantathüllenfertigungsform 3 senkrecht zur Längsachse L. Die Querschnittsweite der Rückseite 35 ist durch den Außenumfang des Übergangs bestimmt. Die Querschnittsweite der Rückseite 35 kann bei einer Implantathüllenfertigungsform 3 mit kreisrundem Querschnitt senkrecht zur Längsachse L durch deren konstanten Kreisdurchmesser definiert sein. Im Rahmen der Erfindung sind auch andere Implantathüllenfertigungsformen denkbar, beispielsweise solche mit eiförmigem oder ovalem Querschnitt senkrecht zur Längsachse L, wobei die kleinste Querschnittsweite beispielsweis bei einem ovalen oder eiförmigen Querschnitt durch die kürzeste Diagonale definiert ist.

Weiterhin kann die Auflageeinheit 8, insbesondere mithilfe der Fixiervorrichtung, gegen die Rückseite 35 der Implantathüllenfertigungsform 3 gedrängt werden. Abschließend wird die mit dem Kit-of-Parts-Werkzeugsatz 1 gehaltene Struktur aus der Silikonbasishülle 101, der wenigstens einen zusätzlichen unverfestigten Silikonschicht 103 und der Schaumlage 105 zumindest einem Aushärtungsschritt unterzogen, um das unausgehärtete Silikon zu Vulkanisieren und um eine innige Verbindung der Schaumlage 105 mit der Implantathülle 100 herbeizuführen. Auf diese Weise wird eine Implantathülle 100 gebildet, die fest, insbesondere materialschlüssig, vorzugsweise integral, mit einer Schaumlage 105 verbunden ist.

Anschließend kann überschüssiger Schaumlage, die nicht durch diesen Aushärtungsschritt mit der Rückseite der Implantathülle 100 verbunden wurde, abgetrennt werden, beispielsweise durch einen Stanzvorgang. Vor der Verwendung der Implantathülle 100 wird diese außerdem von der Implantathüllenfertigungsform 3 entfernt.

Die mit dem Kit-of-Parts-Werkzeugsatz 1 herstellbare oder hergestellte Implantathülle 100 dehnt sich unterbrechungsfrei über sowohl die gesamte Vorderseite 31 wie auch den gesamten Übergang 33 zwischen Vorderseite 31 und Rückseite 35 und die gesamte Rückseite 35 mit Ausnahme des Zentrums 30 aus. Die Implantathülle 100 weist in dem fertigen Zustand eine einzige Öffnung im Bereich des Zentrums 30 an der Rückseite 35 auf und umschließt die Implantathüllenfertigungsform 3 im Übrigen vollständig, naht- und unterbrechungsfrei. Vorzugsweise bilden auch die einzelnen Komponenten der Implantathülle, also die Silikonbasishülle 101, die zusätzliche Silikonschicht 103 und/oder die Schaumlage 105 (jeweils für sich genommen) eine unterbrechungsfreie und/oder nahtfreie, mit Ausnahme einer einzelnen Öffnung in einem rückseitigen Zentralbereich, vollumfängliche Kapsel. Nach dem Entfernen der Implantathülle 100 von der Implantathüllenfertigungsform 3 weist die Implantathülle 100 rückseitig eine Öffnung (Patchloch) auf, die durch ein Patch verschließbar ist. Vorzugsweise kann die Öffnung eine maximale Querschnittsweite, insbesondere einen Durchmesser, von nicht mehr als 6 cm, insbesondere nicht mehr als 5 cm, vorzugsweise nicht mehr als 4 cm, aufweisen und/oder von wenigstens 1 cm, insbesondere wenigstens 2 cm, vorzugsweise wenigstens 3 cm.

Die in der voranstehenden Beschreibung und den Ansprüchen offenbarten Merkmale der Erfindung können sowohl einzeln als auch in jeder beliebigen Kombination für die Verwirklichung der Erfindung in ihren verschiedenen Ausführungsformen wesentlich sein.

### Bezugszeichenliste

- 1: Kit-of-Parts-Werkzeugsatz
- 3: Implantathüllenfertigungsform
- 31: Vorderseite
- 33: Übergang
- 35: Rückseite
- 30: Zentrum
- 5: Haltestab
- 50: Durchgangs- oder Aufnahmekanal
- 51: erstes Ende
- 59: zweites Ende
- 6: Ringstruktur
- 8: Auflageeinheit
- 80: erster Endabschnitt
- 81: umlaufende Wandfläche
- 83: Anlagefläche
- 89: zweiter Endabschnitt
- 9: Fixierstab
- 100: Implantathülle / Brustimplantathülle
- 101: Silikonbasishülle
- 103: unausgehärtete Silikonschicht
- 105: Schaumstofflage
- L: Längsachse
- M: Mittelachse
- α: spitzer Winkel

## Patentansprüche

1. Kit-of-Parts-Werkzeugsatz (1) für die Herstellung von mit einer Schaumlage (105) versehenen Implantathüllen (100) auf Silikonbasis, umfassend
a) eine Implantathüllenfertigungsform (3) mit einer Vorderseite (31), einer Rückseite (35) und einem Übergang (33) von Vorder- zu Rückseite sowie mit einer rückseitig vorliegenden Anbindungseinheit für eine Haltevorrichtung (5),
b) mindestens eine Haltevorrichtung (5) mit einem ersten und einem gegenüberliegenden zweiten Ende (51, 59) mit einer Längsachse und mit einer zu der Anbindungseinheit der Implantathüllenfertigungsform (3) korrespondierenden Anbindungseinheit an dem ersten Ende (51),
c) eine Auflageeinheit (8) mit einem ersten Endabschnitt (80) mit einer umlaufenden Wandfläche (81) und einem gegenüberliegenden zweiten Endabschnitt (89) und mit einer Anlagefläche (83) an dem ersten Endabschnitt (80), ausgelegt und eingerichtet, um in einem Areal um die rückseitig vorliegende Anbindungseinheit anlegbar zu sein, wobei die umlaufende Wandfläche (81) des ersten Endabschnitts (80) der Auflageeinheit (8) bei Anlage der Anlagefläche (83) in einem Übergangsbereich zwischen der Rückseite (35) und dem ersten Endabschnitt eine radiale Einbuchtung bildet,
d) eine Fixiervorrichtung(9) für die temporäre Fixierung der Auflageeinheit (8) an der Rückseite (35) der Implantathüllenfertigungsform (3)
und
e) eine in die radiale Einbuchtung einsetzbare oder eingesetzte Ringstruktur (6),
oder umfassend
i) eine Implantathüllenfertigungsform (3) mit einer Vorderseite (31), einer Rückseite (35) und einem Übergang (33) von Vorder- zu Rückseite sowie mit einer rückseitig vorliegenden Haltevorrichtung (5) mit einer Längsachse,
ii) eine Auflageeinheit (8) mit einem ersten Endabschnitt (80) mit einer umlaufenden Wandfläche (81) und einem gegenüberliegenden zweiten Endabschnitt (89) und mit einer Anlagefläche (83) an dem ersten Endabschnitt (80), ausgelegt und eingerichtet, um in einem Areal um die rückseitig vorliegende Haltevorrichtung anlegbar zu sein, wobei die umlaufende Wandfläche (81) des ersten Endabschnitts (80) der Auflageeinheit (8) bei Anlage der Anlagefläche (83) an dem ersten Endabschnitt (80) an die Rückseite (35) der Implantathüllenfertigungsform (3) in einem Übergangsbereich zwischen der Rückseite (35) und dem ersten Endabschnitt eine radiale Einbuchtung bildet,
iii) eine Fixiervorrichtung (9) für die temporäre Fixierung der Auflageeinheit (8) an der Rückseite (35) der Implantathüllenfertigungsform (3)
und
iv) eine in die Einbuchtung einsetzbare oder eingesetzte Ringstruktur (6).

2. Kit-of-Parts-Werkzeugsatz (1) nach Anspruch 1, **dadurch gekennzeichnet, dass** die Rückseite (35) der Implantathüllenfertigungsform (3) flach ist oder sich, insbesondere zum Zentrum (30) derselben, auswölbt.

3. Kit-of-Parts-Werkzeugsatz (1) nach Anspruch 1 oder 2, **dadurch gekennzeichnet, dass**
die Rückseite (35) der Implantathüllenfertigungsform (3) kegelförmig oder kegelstumpfförmig ausgebildet ist.

4. Kit-of-Parts-Werkzeugsatz (1) nach einem der vorangehenden Ansprüche, **dadurch gekennzeichnet, dass**
die Auflageeinheit (8) jenseits des ersten Endabschnitts (80) im Wesentlichen zylinderförmig ausgebildet ist.

5. Kit-of-Parts-Werkzeugsatz (1) nach einem der vorangehenden Ansprüche, **dadurch gekennzeichnet, dass** die Ringstruktur (6) gebildet ist durch eine federelastische, insbesondere bandförmige, Ringstruktur (6), insbesondere eingerichtet und ausgelegt, um zerstörungsfrei über die Implantathüllenfertigungsform (3) führbar und unter Spannung in den spitzen Winkel (α) zwischen der Rückseite (35) und dem ersten Endabschnitt (80) der Auflageeinheit (8) anlegbar zu sein, oder einen reversibel öffenbaren Metallring oder einen biegbaren Draht, eingerichtet und ausgelegt, um in den spitzen Winkel (α) zwischen der Rückseite (35) und dem ersten Endabschnitt (80) der Auflageeinheit (8) unter Ausbildung einer Ringstruktur (6) anlegbar zu sein

6. Kit-of-Parts-Werkzeugsatz (1) nach einem der vorangehenden Ansprüche, **dadurch gekennzeichnet, dass**
die rückseitig vorliegende Anbindungseinheit für eine Haltevorrichtung im Zentrum (30) der Rückseite (35) der Implantathüllenfertigungsform (3) vorliegt.

7. Kit-of-Parts-Werkzeugsatz (1) nach einem der vorangehenden Ansprüche, **dadurch gekennzeichnet, dass**
die rückseitig vorliegende Anbindungseinheit für eine Haltevorrichtung
und die Anbindungseinheit an dem ersten Ende (51) der Haltevorrichtung ausgelegt und eingerichtet sind, eine reversibel lösbare kraftschlüssige und/oder formschlüssige Verbindung einzugehen.

8. Kit-of-Parts-Werkzeugsatz (1) nach einem der vorangehenden Ansprüche, **dadurch gekennzeichnet, dass**
die rückseitig vorliegende Anbindungseinheit für eine Haltevorrichtung eine Gewindebohrung umfasst oder darstellt und dass die korrespondierende Anbindungseinheit an dem ersten Ende (51) der Haltevorrichtung ein, insbesondere passgenaues, Gewinde umfasst oder darstellt oder dass
die rückseitig vorliegende Anbindungseinheit für eine Haltevorrichtung ein Gewinde umfasst oder darstellt und dass die korrespondierende Anbindungseinheit an dem ersten Ende (51) der Haltevorrichtung eine, insbesondere passgenaue, Gewindebohrung umfasst oder darstellt.

9. Kit-of-Parts-Werkzeugsatz (1) nach einem der vorangehenden Ansprüche, **dadurch gekennzeichnet, dass**
die Implantathüllenfertigungsform (3) und/oder die Auflageeinheit (8) Metall, insbesondere Aluminium, oder Kunststoff, insbesondere Polyacetale, Polyethersulfone, Polysulfone, Polyphenylsulfone, Polyetheretherketone, Polyetherketone, thermoplastische Polyimide, Polyphenylensulfide, Polyamide, Polyphenylenether, Polycarbonate, Polyethylenterephthalat und Polybutylenterephthalat, umfasst oder hieraus besteht.

10. Kit-of-Parts-Werkzeugsatz (1) nach einem der vorangehenden Ansprüche, **dadurch gekennzeichnet, dass**
die Implantathüllenfertigungsform (3) eine Brustimplantathüllenfertigungsform darstellt.

11. Kit-of-Parts-Werkzeugsatz (1) nach einem der vorangehenden Ansprüche, **dadurch gekennzeichnet, dass**
der Abstand zwischen dem ersten und dem zweiten Endabschnitt (89) der Auflageeinheit (8) im Wesentlichen dem Abstand zwischen dem Durchgangs- oder Aufnahmekanal und der Anbindungseinheit an dem ersten Ende (51) der Haltevorrichtung entspricht.

12. Kit-of-Parts-Werkzeugsatz (1) nach einem der vorangehenden Ansprüche, **dadurch gekennzeichnet, dass**
die Haltevorrichtung (5) einen Haltestab darstellt.

13. Kit-of-Parts-Werkzeugsatz (1) nach einem der vorangehenden Ansprüche, **dadurch gekennzeichnet, dass**
die mindestens eine Haltevorrichtung (5) einen Durchgangs- oder Aufnahmekanal im Wesentlichen senkrecht zu der Längsachse umfasst.

14. Kit-of-Parts-Werkzeugsatz (1) nach einem der vorangehenden Ansprüche, **dadurch gekennzeichnet, dass**
die Anlagefläche (83) an dem ersten Endabschnitt (80) ausgelegt und eingerichtet ist, um in einem Areal um die rückseitig vorliegende Anbindungseinheit bündig anlegbar zu sein.

15. Kit-of-Parts-Werkzeugsatz (1) nach einem der vorangehenden Ansprüche, **dadurch gekennzeichnet, dass**
die radiale Einbuchtung an dem ersten Endabschnitt (80) an der Rückseite (35) der Implantathüllenfertigungsform (3) einen spitzen Winkel (α) bildet.

16. Kit-of-Parts-Werkzeugsatz (1) nach einem der vorangehenden Ansprüche, **dadurch gekennzeichnet, dass**
die Auflageeinheit (8) sich am ersten Endabschnitt (80) in Richtung der Anlagefläche (83) verjüngt.

17. Kit-of-Parts-Werkzeugsatz (1) nach einem der vorangehenden Ansprüche, **dadurch gekennzeichnet, dass**
die Fixiervorrichtung (9) einen Fixierstab darstellt.

18. Kit-of-Parts-Werkzeugsatz (1) nach einem der vorangehenden Ansprüche, **dadurch gekennzeichnet, dass**
die Fixiervorrichtung (9) einen Durchmesser aufweist, der kleiner ist als der Durchmesser des Durchgangs- oder Aufnahmekanals, und eine Länge aufweist, die gleich oder größer ist als der Durchmesser des zweiten Endabschnitts (89) der Auflageeinheit (8).

## Claims

1. Kit-of-parts tool kit (1) for manufacturing silicone-based implant shells (100) provided with a foam layer (105), comprising
a) an implant shell production mold (3) with a front side (31), a back side (35), and a transition (33) from the front side to the back side, as well as a connection unit on the back side for a holding device (5),
b) at least one holding device (5), with a first and an opposite second end (51, 59) with a longitudinal axis and with a connection unit at the first end (51) corresponding to the connection unit of the implant shell production mold (3),
c) a support unit (8) with a first end section (80) with a circumferential wall surface (81) and an opposite second end section (89) and with a contact surface (83) on the first end section (80), designed and configured to be placed in an area around the rear connection unit, wherein the circumferential wall surface (81) of the first end section (80) of the support unit (8) forms a radial indentation when the abutment surface (83) abuts in a transition area between the back side (35) and the first end section,
d) a fixing device (9), for temporarily fixing the support unit (8) to the back side (35) of the implant shell production mold (3),
and
e) a ring structure (6) that can be inserted or is inserted into the radial indentation,
or comprising
i) an implant shell production mold (3) with a front side (31), a back side (35) and a transition (33) from the front side to the back side, as well as a holding device (5) located on the back side with a longitudinal axis,
ii) a support unit (8) with a first end section (80) with a circumferential wall surface (81) and an opposite second end section (89) and with a contact surface (83) on the first end section (80), designed and configured to be placed in an area around the holding device located at the back, wherein the circumferential wall surface (81) of the first end section (80) of the support unit (8) forms a radial indentation in a transition area between the back side (35) and the first end section when the abutment surface (83) on the first end section (80) abuts against the back side (35) of the implant shell production mold (3) in a transition area between the back side (35) and the first end section,
iii) a fixing device, in particular a fixing rod (9), for temporarily fixing the support unit (8) to the back side (35) of the implant shell production mold (3),
and
iv) a ring structure (6) that can be inserted or is inserted into the indentation.

2. Kit-of-parts tool set (1) according to claim 1, **characterized in that** the back side (35) of the implant shell production mold (3) is flat or bulges in particular toward the center (30) thereof.

3. Kit-of-parts tool set (1) according to claim 1 or 2, **characterized in that** the back side (35) of the implant shell production mold (3) is conical or truncated conical in shape.

4. Kit-of-parts tool set (i) according to one of the preceding claims, **characterized in that** the support unit (8) beyond the first end section (80) is essentially cylindrical in shape.

5. Kit-of-parts tool set (1) according to one of the preceding claims, **characterized in that** the ring structure (6) is formed by a spring-elastic, in particular tape-shaped, ring structure (6), in particular arranged and designed to be guided non-destructively over the implant shell production mold (3) and to be applied under tension in the acute angle (α) between the back side (35) and the first end section (80) of the support unit (8), or a reversibly openable metal ring or a bendable wire, arranged and designed to be applied in the acute angle (α) between the back side (35) and the first end section (80) of the support unit (8) to form a ring structure (6).

6. Kit-of-parts tool set (1) according to one of the preceding claims, **characterized in that** the rear connection unit for a holding device is located in the center (30) of the back side (35) of the implant shell production mold (3).

7. Kit-of-parts tool set (1) according to one of the preceding claims, **characterized in that** the connection unit on the back side for a holding device and the connection unit at the first end (51) of the holding device are designed and configured to form a reversibly detachable force-fit and/or form-fit connection.

8. Kit-of-parts tool set (1) according to one of the preceding claims, **characterized in that** the connection unit for a holding device located on the back side comprises or represents a threaded bore and that the corresponding connection unit at the first end (51) of the holding device comprises or represents a thread, in particular a precisely fitting thread, or that
the rear connection unit for a holding device comprises or represents a thread and that the corresponding connection unit at the first end (51) of the holding device comprises or represents a threaded hole, in particular a precisely fitting one.

9. Kit-of-parts tool set (1) according to one of the preceding claims, **characterized in that** the implant shell production mold (3) and/or the support unit (8) comprise metal, in particular aluminum, or plastic, in particular polyacetals, preferably polyoxymethylene, polyethersulfones, polysulfones, polyphenylsulfones, polyetheretherketones, polyetherketones, thermoplastic polyimides, polyphenylenesulfides, polyamides, poly polyphenylenethers, polycarbonates, polyethylene terephthalate, and polybutylene terephthalate.

10. Kit-of-parts tool kit (1) according to one of the preceding claims, **characterized in that** the implant shell production mold (3) is a breast implant shell production mold.

11. Kit-of-parts tool set (1) according to one of the preceding claims, **characterized in that** the distance between the first and the second end section (89) of the support unit (8) essentially corresponds to the distance between the passage or receiving channel and the connection unit at the first end (51) of the holding device.

12. Kit-of-parts tool set (1) according to one of the preceding claims, **characterized in that** the holding device (5) comprises a holding rod.

13. Kit-of-parts tool set (1) according to one of the preceding claims, **characterized in that** the at least one holding device (5) comprises a through-hole or receiving channel substantially perpendicular to the longitudinal axis.

14. Kit-of-parts tool set (1) according to one of the preceding claims, **characterized in that** the abutment surface (83) on the first end section (80) is designed and configured to lie flush in an area around the rear connection unit.

15. Kit-of-parts tool set (1) according to one of the preceding claims, **characterized in that** the radial indentation on the first end section (80) forms an acute angle (α) with the rear surface (35) of the implant shell production mold (3).

16. Kit-of-parts tool set (1) according to one of the preceding claims, **characterized in that** the support unit (8) tapers at the first end section (80) towards the abutment surface (83).

17. Kit-of-parts tool set (1) according to one of the preceding claims, **characterized in that** the fixing device (9) comprises a fixing rod.

18. Kit-of-parts tool set (1) according to one of the preceding claims, **characterized in that** the fixing device (9) has a diameter which is smaller than the diameter of the through-hole or receiving channel, and has a length which is equal to or greater than the diameter of the second end section (89) of the support unit (8).

## Revendications

1. Jeu d'outils (1) pour la fabrication de coques d'implants à base de ksilicone (100) pourvues d'une couche de mousse (105), comprenant
a) un moule de fabrication d'enveloppe d'implant (3) avec une face avant (31), une face arrière (35) et une transition (33) entre la face avant et la face arrière, ainsi qu'une unité de raccordement sur la face arrière pour un dispositif de maintien (5),
b) au moins un dispositif de maintien (5), comportant une première extrémité et une deuxième extrémité opposée (51, 59) avec un axe longitudinal et une unité de raccordement à la première extrémité (51) correspondant à l'unité de raccordement du moule de fabrication d'enveloppes d'implants (3),
c) une unité de support (8) comportant une première section d'extrémité (80) avec une surface de paroi circonférentielle (81) et une deuxième section d'extrémité opposée (89), ainsi qu'une surface de contact (83) sur la première section d'extrémité (80), conçue et configurée pour être placée dans une zone autour de l'unité de raccordement arrière, dans laquelle la surface de paroi circonférentielle (81) de la première section d'extrémité (80) de l'unité de support (8) forme une indentation radiale lorsque la surface d'appui (83) vient en butée dans une zone de transition entre la face arrière (35) et la première section d'extrémité,
d) un dispositif de fixation (9), destiné à fixer temporairement l'unité de support (8) à la face arrière (35) du moule de fabrication de l'enveloppe d'implant (3), et
e) une structure annulaire (6) pouvant être insérée ou étant insérée dans l'indentation radiale,
ou comprenant
i) un moule de fabrication de coque d'implant (3) avec une face avant (31), une face arrière (35) et une transition (33) entre la face avant et la face arrière, ainsi qu'un dispositif de maintien (5) situé sur la face arrière et présentant un axe longitudinal,
ii) une unité de support (8) comportant une première section d'extrémité (80) avec une surface de paroi circonférentielle (81) et une deuxième section d'extrémité opposée (89), ainsi qu'une surface de contact (83) sur la première section d'extrémité (80), conçue et configurée pour être placée dans une zone autour du dispositif de maintien situé à l'arrière, dans laquelle la surface de paroi circonférentielle (81) de la première section d'extrémité (80) de l'unité de support (8) forme une indentation radiale dans une zone de transition entre la face arrière (35) et la première section d'extrémité lorsque la surface d'appui (83) de la première section d'extrémité (80) vient en butée contre la face arrière (35) du moule de fabrication de l'enveloppe d'implant (3) dans une zone de transition entre la face arrière (35) et la première section d'extrémité,
iii) un dispositif de fixation, en particulier une tige de fixation (9), destiné à fixer temporairement l'unité de support (8) à la face arrière (35) du moule de fabrication de la coque d'implant (3),
et
iv) une structure annulaire (6) pouvant être insérée ou étant insérée dans l'indentation.

2. Jeu d'outils (1) selon la revendication 1, **caractérisé en ce que** la face arrière (35) du moule de fabrication de coques d'implants (3) est plate ou présente un renflement, en particulier vers son centre (30).

3. Jeu d'outils (1) selon la revendication 1 ou 2, **caractérisé en ce que** la face arrière (35) du moule de fabrication de coque d'implant (3) a une forme conique ou tronconique.

4. Jeu d'outils (1) selon l'une des revendications précédentes, **caractérisé en ce que** l'unité de support (8) au-delà de la première section d'extrémité (80) a une forme essentiellement cylindrique.

5. Jeu d'outils (1) selon l'une des revendications précédentes, **caractérisé en ce que** la structure annulaire (6) est formée par une structure annulaire (6) élastique, en particulier en forme de bande, en particulier agencée et conçue pour être guidée de manière non destructive sur le moule de fabrication de l'enveloppe d'implant (3) et pour être appliquée sous tension dans l'angle aigu (α) entre la face arrière (35) et la première section d'extrémité (80) de l'unité de support (8), ou un anneau métallique pouvant s'ouvrir de manière réversible ou un fil métallique pliable, agencé et conçu pour être appliqué dans l'angle aigu (α) entre la face arrière (35) et la première section d'extrémité (80) de l'unité de support (8) afin de former une structure annulaire (6).

6. Jeu d'outils (1) selon l'une des revendications précédentes, **caractérisé en ce que** l'unité de connexion arrière pour un dispositif de maintien est située au centre (30) de la face arrière (35) du moule de fabrication de la coque d'implant (3).

7. Jeu d'outils (1) selon l'une des revendications précédentes, **caractérisé en ce que** l'unité de raccordement sur la face arrière pour un dispositif de maintien et l'unité de raccordement à la première extrémité (51) du dispositif de maintien sont conçues et configurées pour former une liaison par ajustement serré et/ou par complémentarité de forme, amovible de manière réversible.

8. Jeu d'outils (1) selon l'une des revendications précédentes, **caractérisé en ce que** l'unité de raccordement pour un dispositif de maintien située sur la face arrière comprend ou représente un alésage fileté et **en ce que** l'unité de raccordement correspondante à la première extrémité (51) du dispositif de maintien comprend ou représente un filetage, en particulier un filetage à ajustement précis, ou **en ce que**
l'unité de raccordement arrière d'un dispositif de fixation comprend ou constitue un filetage et que l'unité de raccordement correspondante à la première extrémité (51) du dispositif de fixation comprend ou constitue un trou fileté, en particulier un trou à ajustement précis.

9. Jeu d'outils (1) selon l'une des revendications précédentes, **caractérisé en ce que** le moule de fabrication de l'enveloppe d'implant (3) et/ou l'unité de support (8) sont constitués de métal, en particulier d'aluminium, ou de plastique, en particulier de polyacétals, de préférence de polyoxyméthylène, polyéthersulfones, polysulfones, polyphénylsulfones, polyétheréthercétones, polyéthercétones, polyimides thermoplastiques, polyphénylènesulfures, polyamides, polyphénylèneéthers, polycarbonates, polyéthylène téréphtalate et polybutylène téréphtalate.

10. Trousse à outils (1) selon l'une des revendications précédentes, **caractérisée en ce que** le moule de fabrication d'enveloppe d'implant (3) est un moule de fabrication d'enveloppe d'implant mammaire.

11. Ensemble d'outils (1) selon l'une des revendications précédentes, **caractérisé en ce que** la distance entre la première et la deuxième section d'extrémité (89) de l'unité de support (8) correspond essentiellement à la distance entre le passage ou le canal de réception et l'unité de raccordement à la première extrémité (51) du dispositif de maintien.

12. Jeu d'outils (1) selon l'une des revendications précédentes, **caractérisé en ce que** le dispositif de maintien (5) comprend une tige de maintien.

13. Jeu d'outils (1) selon l'une des revendications précédentes, **caractérisé en ce que** le au moins un dispositif de maintien (5) comprend un trou traversant ou un canal de réception sensiblement perpendiculaire à l'axe longitudinal.

14. Jeu d'outils (1) selon l'une des revendications précédentes, **caractérisé en ce que** la surface d'appui (83) sur la première section d'extrémité (80) est conçue et configurée pour affleurer dans une zone autour de l'unité de raccordement arrière.

15. Jeu d'outils (1) selon l'une des revendications précédentes, **caractérisé en ce que** l'indentation radiale sur la première section d'extrémité (80) forme un angle aigu (α) avec la surface arrière (35) du moule de fabrication de l'enveloppe d'implant (3).

16. Jeu d'outils (1) selon l'une des revendications précédentes, **caractérisé en ce que** l'unité de support (8) se rétrécit au niveau de la première section d'extrémité (80) en direction de la surface d'appui (83).

17. Ensemble d'outils (1) selon l'une des revendications précédentes, **caractérisé en ce que** le dispositif de fixation (9) comprend une tige de fixation.

18. Jeu d'outils (1) selon l'une des revendications précédentes, **caractérisé en ce que** le dispositif de fixation (9) a un diamètre inférieur au diamètre du trou traversant ou du canal de réception, et a une longueur égale ou supérieure au diamètre de la deuxième section d'extrémité (89) de l'unité de support (8).
